# EUROPEAN PATENT APPLICATION

(11) **EP 4 759 352 A1**
(43) Date of publication of application: **17.06.2026**
(21) Application number: 25219648.0
(22) Date of filing: 01.12.2025
(51) Int. Cl.: A61N 1/36, A61N 1/37, A61N 1/39, A61N 1/372

(54) **SYSTEM FOR DISCRIMINATING BETWEEN MAGNETIC FIELDS USING AN IMPLANTED MEDICAL DEVICE**

(30) Priority: 12.12.2024 US 202463733091 P; 31.10.2025 US 202519376317
(71) Applicant: Pacesetter, Inc., Sylmar, CA 91342 (US)
(72) Inventor: Childers, Michael, Sylmar, CA 91342 (US); Sison, Shiloh, Sylmar, CA 91342 (US); Feng, Shi, Sylmar, CA 91342 (US); Vardhan Tiwari, Yash, Sylmar, CA 91342 (US); Jiang, Ruoli, Sylmar, CA 91342 (US); Pei, Xing, Sylmar, CA 91342 (US); Bornzin, Alexander R, Sylmar, CA 91342 (US)
(74) Representative: Barker Brettell Sweden AB

(57) **Abstract**

An implantable medical device (IMD) (200) is provided that can include electronic circuitry (212), a first sensor (270) configured to detect a magnetic field in an environment of the IMD (200), and a second sensor (110, 112, 114, 234) configured to obtain environmental data. The IMD (200) includes one or more processors (264), and a memory (252) coupled to the one or more processors (264). The memory (252) stores program instructions, wherein the program instructions are executable by the one or more processors (264) to discriminate between a first magnet mode and a second magnet mode based on the environmental data and switch the mode of operation with the electronic circuitry (212) based on discriminating between the first magnet mode and the second magnet mode.

## Description

### TECHNICAL FIELD

Embodiments herein generally relate to systems or methods for detecting magnetic fields using an active implanted medical device (IMD).

### BACKGROUND

An IMD is a medical device that is configured to be implanted within a patient anatomy and commonly employs one or more leads with electrodes that either receive or deliver voltage, current or other electromagnetic pulses from or to an organ or tissue for diagnostic or therapeutic purposes. In general, IMDs include a battery, electronic circuitry, a pulse generator, a transceiver and/or a microprocessor that is configured to manage communication with an external instrument as well as control patient therapy. The components of the IMD are hermetically sealed within a metal housing.

When a patient with an active IMD undergoes a magnetic resonance (MR) scan, sensing and high voltage (HV) therapy are disabled due to the interference signals generated by the MR scanner. Interference is especially true of the signals generated by the switching gradients because the frequency content is similar to cardiac activity signals. In order to discriminate between MR generated and cardiac activity signals, accurate acquisition of the MRI generated signals is required.

Existing MRI scanning workflow requires manually programming the device into and out of an MRI mode. This is a burden on the field staff and results in patients being in suboptimal pacing mode for longer than would otherwise be necessary. For example, staff may forget to manually switch the mode of operation of the IMD into the MRI mode resulting in incorrect therapy.

As a solution to prevent clinician and technicians from forgetting to manually switch an IMD into an MRI mode, MRI autodetection exists. Problematically many IMDs also account for the detection of a magnet field caused by handheld magnets in an environment by automatically switching the IMD into a magnet mode. Unfortunately, the static magnetic field level generated by an MRI scanner and that generated by an environmental magnet, such as a handheld magnet, can overlap. This may lead to an IMD, such as an implanted pacemaker, implantable cardioverter defibrillator (ICD) or cardiac resynchronization therapy (CRT) device, with an MRI autodetect feature to transition into the magnet mode in the presence of an MRI static field. Similarly, this may lead to an implanted pacemaker, ICD or CRT device with an MRI autodetect feature to transition into MRI mode in the presence of an environmental magnet. Because the system parameters differ between the MRI mode and magnet mode, there is a need to discriminate between the non-MRI environment where an environmental magnet is expected and an MRI scanner environment where time-varying radio frequency (RF) and gradient electromagnetic fields are expected.

### SUMMARY

In accordance with embodiments herein, an implantable medical device (IMD) is provided that can include electronic circuitry, a first sensor configured to detect a magnetic field in an environment of the IMD, and a second sensor configured to obtain environmental data. The IMD also includes one or more processors, and a memory coupled to the one or more processors. The memory stores program instructions, wherein the program instructions are executable by the one or more processors to discriminate between a first magnet mode and a second magnet mode based on the environmental data and switch the mode of operation with the electronic circuitry based on discriminating between the first magnet mode and the second magnet mode.

Optionally, the program instructions can be executable by the one or more processors to operate the second sensor with the circuitry in response to detecting the magnetic field.

Optionally, the program instructions are executable by the one or more processors to discriminate between the first magnet mode and the second magnet mode by identifying noise caused by RF and/or gradient electromagnetic field based on the environmental data.

Optionally, the second sensor may include at least one lead of the IMD that is configured to obtain a cardiac activity signal and the noise is within the cardiac activity signal.

Optionally, the IMD can be a leadless device that includes a component configured to obtain a cardiac activity signal and the noise is within the cardiac activity signal.

Optionally, the program instructions can be executable by the one or more processors to verify the noise within the cardiac activity signal is in response to the RF and/or gradient electromagnetic field.

Optionally, the program instructions are executable by the one or more processors to verify the noise within the cardiac activity signal by determining whether the noise is within at least two noise windows of the cardiac activity signal.

Optionally, the second sensor may include telemetry circuitry configured to transmit communication signals exterior to the IMD and wherein the noise is detected within the communication signals.

Optionally, the program instructions are executable by the one or more processors to determine the noise by opening, with the telemetry circuitry, a communication pathway for a determined amount of time, digitizing at least one communication packet traveling through the communication pathway, determining that the at least one communication packet is an invalid communication packet, and identifying the noise based on the invalid communication packet determination.

Optionally, the program instructions can be executable by the one or more processors to operate the IMD in an automatic MRI mode wherein the first sensor is configured to detect the magnetic field in an environment of the IMD. Preferably, the automatic MRI mode can include the first magnet mode and the second magnet mode and the program instructions are executable by the one or more processors to operate the IMD in the first magnet mode in response to the first sensor detecting an initial threshold has been exceeded and operate in the second magnet mode when a secondary threshold has been exceeded.

Optionally, the first magnet mode can include a first setting based on the IMD being in close proximity with an environmental magnet, and the second magnet mode includes a second setting based on the IMD being within an MRI machine. In one example, the initial threshold can be 15 Gauss, and the secondary threshold is greater than the initial threshold. In another example, the secondary threshold can vary in value based on time operating in the first magnet mode.

Optionally, the program instructions can be executable by the one or more processors to treat a patient based on the first setting when the IMD is within the first magnet mode and treat the patient based on the second setting when the IMD is within the second magnet mode.

Optionally, the IMD may include a non-magnetic operation mode.

Optionally, the program instructions can be executable by the one or more processors to switch the IMD into the first magnet mode in response to detecting the magnetic field, maintain the IMD in the first magnet mode in response to detecting the magnetic field is generated by an environmental magnet, and switch the IMD into the second magnet mode in response to determining the magnetic field is generated by a magnetic resonance imaging (MRI) machine.

Optionally, the program instructions can be executable by the one or more processors to switch the IMD to a non-magnetic operating mode in response to determining the magnetic field has been removed from the environment of the IMD.

Optionally, to discriminate between the first magnet mode and the second magnet mode the program instructions can be executable by the one or more processors to determine a strength of the magnetic field detected by the first sensor, and operate the IMD in the first magnet mode when the strength of the magnetic field is below a magnetic field threshold and operate the IMD in the second magnet mode when the strength of the magnetic field is at or above the magnetic field threshold.

Optionally, the first sensor can be at least one of a Hall effect sensor, a three-axis Hall effect sensor, or giant magnetoresistance sensor.

In accordance with embodiments herein, a method is provided that can include, under control of one or more processors, detecting, with a first sensor, a magnetic field in an environment of an implantable medical device (IMD). The method also includes discriminating between a first magnet mode and a second magnet mode based on environmental data obtained from a second sensor and switching the mode of operation with electronic circuitry based on discriminating between the first magnet mode and the second magnet mode.

Optionally, the method can also include operating the second sensor with the electronic circuitry in response to detecting the magnetic field.

Optionally, discriminating between the first magnet mode and the second magnet mode can include identifying noise caused by the RF and/or gradient electromagnetic field based on the environmental data.

Optionally, the discriminating between the first magnet mode and the second magnet mode can include obtaining, with the second sensor a cardiac activity signal and the noise is within the cardiac activity signal.

Optionally, the method can additionally include verifying the noise within the cardiac activity signal is in response to the RF and/or gradient electromagnetic field.

Optionally, verifying the noise within the cardiac activity signal may include determining whether the noise is within at least two noise windows of the cardiac activity signal.

Optionally, discriminating between the first magnet mode and the second magnet mode can include opening, with telemetry circuitry of the second sensor, a communication pathway for a determined amount of time, digitizing at least one communication packet traveling through the communication pathway, determining that the at least one communication packet is an invalid communication packet, and identifying the noise based on the invalid communication packet determination.

Optionally, the method can also include treating a patient based on a first setting when the IMD is within the first magnet mode and treating the patient based on a second setting when the IMD is within the second magnet mode.

Optionally, the method can also include switching the IMD into the first magnet mode in response to detecting the magnetic field, maintaining the IMD in the first magnet mode in response to detecting the magnetic field is generated by an environmental magnet, and switching the IMD into the second magnet mode in response to determining the magnetic field is generated by a magnetic resonance imaging (MRI) machine.

Optionally, the switching the IMD to a non-magnetic operating mode in response to determining the magnetic field may not detected in the environment of the IMD.

Optionally, switching the IMD to a non-magnetic operating mode in response to determining the magnetic field may not detected in the environment of the IMD for a determined period of time.

Optionally, discriminating between the first magnet mode and the second magnet mode can include determining a strength of the magnetic field detected by the first sensor, and operating the IMD in the first magnet mode when the strength of the magnetic field is below a magnetic field threshold and operate the IMD in the second magnet mode when the strength of the magnetic field is at or above the magnetic field threshold.

In accordance with embodiments herein, an implantable medical device (IMD) is provided that can include electronic circuitry, a first sensor configured to detect a magnetic field in an environment of the IMD, and one or more processors. The IMD also includes a memory coupled to the one or more processors, wherein the memory stores program instructions, wherein the program instructions are executable by the one or more processors to switch the IMD into a first magnet mode in response to detecting the magnetic field. The one or more processors are also configured to discriminate between the first magnet mode and a second magnet mode based a strength of the magnetic field, select between the first magnet mode and the second magnet mode based on noise detected by the IMD, and switch a mode of operation with the electronic circuitry from the first magnet mode to the second magnet mode based on the noise detected.

Optionally, in the first magnet mode the IMD can have first settings related to the IMD being in close proximity to an environmental magnet, in the second magnet mode the IMD can have second settings related to the IMD being within a magnetic resonance imaging (MRI) machine, and the program instructions are executable by the one or more processors to treat a patient with the IMD based on the mode of operation.

Optionally, IMD can include at least one lead configured to detect a cardiac activity signal, and the program instructions are executable by the one or more processors to determine the noise detected is within the cardiac activity signal.

Optionally, the program instructions can be executable by the one or more processors to verify the noise within the cardiac activity signal is in response to the RF and/or gradient electromagnetic fields of an MRI scanner.

Optionally, to verify the noise within the cardiac activity signal can include determining whether the noise is within at least two noise windows of the cardiac activity signal.

Optionally, the IMD may include telemetry circuitry configured to transmit communication signals exterior to the IMD; and wherein the noise is within the communication signals.

Optionally, to determine the noise can include opening, with the telemetry circuitry, a communication pathway for a determined amount of time, digitizing at least one communication packet traveling through the communication pathway, determining that the at least one communication packet is an invalid communication packet, and identifying the noise based on the invalid communication packet determination.

### DESCRIPTION OF THE DRAWINGS

Figure 1 illustrates a schematic diagram of an IMD in accordance with embodiments herein.
Figure 2 illustrates a schematic block diagram of an IMD in accordance with embodiments herein.
Figure 3 illustrates a block flow diagram of a method of operating an IMD based on detection of a magnetic field in the environment of the IMD, in accordance with embodiments herein.
Figure 4A illustrates an example electrogram, in accordance with embodiments herein.
Figure 4B illustrates an example electrogram, in accordance with embodiments herein.
Figure 5A illustrates an example electrogram, in accordance with embodiments herein.
Figure 5B illustrates an example electrogram, in accordance with embodiments herein.
Figure 5C illustrates an example electrogram, in accordance with embodiments herein.
Figure 6 illustrates a block flow diagram of a method of detecting a strong magnetic field, in accordance with embodiments herein.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems, devices and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

### Terms

The term "electronic circuitry" refers to any and all electronic devices, parts, traces, etc. used to vary current, voltage, resistance, charge, power, or the like using electricity. Example electronic circuitry includes capacitors, transistors, rectifiers, diodes, transformers, coils, telemetry coils, transformer coils, auxiliary coils, batteries, resistors, switches, or the like.

The terms "cardiac activity signal", "cardiac activity signals", "CA signal" and "CA signals" (collectively "CA signals") are used interchangeably throughout to refer to an analog or digital electrical signal recorded by two or more electrodes positioned subcutaneous, cutaneous, or intracardiac on distal end of transvenous lead or device (leadless pacemaker), etc. where the electrical signals are indicative of cardiac electrical activity. The cardiac activity may be normal/healthy or abnormal/arrhythmic. Non-limiting examples of CA signals include electrocardiogram (ECG) signals collected by cutaneous electrodes, and electrogram (EGM) signals collected by subcutaneous electrodes.

The term "biological signal" shall include signals measured by an IMD within the patient, where the signals are indicative of a cardiac activity characteristic, hemodynamic characteristic and/or body generated analyte. The biological signals are conveyed from the IMD within one or both IMD data sets and blood gas analyte (BGA) data sets. Non-limiting examples of biological signals include cardiac activity signals, cardiac impedance, pulmonary impedance, transthoracic impedances, accelerometer signatures, heart sounds, pulmonary arterial pressure signals, blood pressure, rpm levels, flow rates and the like.

The term "cardiac treatment" refers to any and all actions undertaken to treat a medical condition of the heart. Cardiac treatment includes shocks, low voltage (LV) shocks, medium voltage (MV) shocks, high voltage (HV) shocks, pacing, high-frequency (HF) pacing, the delivery of medicine, cardiac resynchronization therapy, or the like, including from an implantable cardioverter defibrillator (ICD).

The terms "physiologic condition" and "pathophysiologic condition" are used to distinguish between a normal/healthy state of a patient condition of interest and an abnormal/unhealthy state of the patient's condition of interest. Non-limiting examples of patient conditions of interest include electrical or hemodynamic cardiac behavior (e.g. normal sinus rhythms, arrhythmias, unstable hemodynamic performance), neurological behavior (e.g. pain, tremors, Parkinson's disease, tinnitus, Alzheimer's disease, and other neurological disorders measurable and treatable within the spine, brain and peripheral muscles), blood pressure, pulse oximetry levels, diabetes and other medical conditions due to imbalances/deficiencies of body generated analytes.

The term "environmental data" refers to any and all information, characteristics, parameters, measurements, etc. that can be detected, sensed, or the like within a physical region where an IMD is located. By way of example, an environment may refer to one or more rooms within a home, office, clinic, hospital, doctors' office, or other structure. An environment may or may not have physical boundaries. For example, an environment instead be defined based upon a range over which a sensor can detect information, characteristics, parameters, measurements, etc. When an IMD is moved an environment associated with the IMD shifts over time. For example, an environment surrounding an IMD moves with the patient with the IMD. An environment surrounding an IMD shift each time the IMD is relocated, such as when moved between different rooms of a hospital, clinicians' office, from indoors to outdoors, or the like. In example embodiments environmental data can include data obtained that indicates the existence of a magnetic field in the environment. The magnetic field can be a static magnetic field, an RF and/or gradient electromagnetic field, or the like.

The term "environmental magnet" refers to any object, structure, or the like that is within an environment of an IMD and generates a weak magnetic field. Examples of environmental magnets include handheld magnets, bar magnets, ring magnets, or the like.

The term "sensor" refers to any device, circuitry, circuit, or the like that can detect, measure, responds to, records, indicates, etc. a physical or electrical property. In one example a sensor can be a first sensor that directly detects a static magnetic field in an environment. In another example a sensor can be a second sensor including a lead, telemetry circuit, or the like that responds to a RF and/or gradient electromagnetic field in an environment.

The term "strong magnetic field" refers to any magnetic field that is greater than 200 G. For the avoidance of doubt, any and all magnetic fields generated by an MRI machine is considered a strong magnetic field as described herein.

The term "weak magnetic field" refers to any magnetic field that is equal to or less than 200 G. Example structures that generate week magnetic fields include handheld magnets, bar magnets, ring magnets, or the like.

The term "magnet mode" refers to a state of operation of a system, assembly, device, etc. that accounts for a magnetic field in an environment. The settings, parameters, or the like of the magnet mode are provided to account for a magnetic field in the environment. It is possible to have multiple magnet modes, for example The first magnet mode may be associated with a handheld magnet environment, while a second magnet mode may be associated with those associated with an MRI magnet environment. In one example, the settings or parameters of an IMD can be related to cardiac treatments, shock treatments, pacing treatments, monitoring, or the like.

The term "gradient electromagnetic field" refers to a time varying switching magnetic field generated by a magnet, machine, device, etc. within an environment. In one example, the gradient electromagnetic field can be generated by an MRI machine when the MRI machine creates the variation needed for spatially encoding an MRI signal.

The term "noise" refers to unwanted signals that interfere with a desired signal. In one example, the desired signal can be a cardiac activity signal including one in an EGM. In another example, the desired signal can be a communication signal received by a telemetry circuit.

The term "noise window" refers to an interval or period of time of a determined duration when a sensor detects noise. For example, the determined duration can be the length of time for blood to cycle through the heart. As an example, when the determined duration is 250 ms, four separate 62.5 ms noise windows can exist during the determined duration. Each individual 62.5 ms time period is considered a separate noise window.

The term "mode" refers to a state of operation of a system, assembly, device, etc. In an example, a first mode of a system is provided when electronic circuitry functions in a first manner, and a second mode is provided when the electronic circuitry operates in a second manner. A first mode and a second mode can have similar functionalities, though some function is different. For example, a telemetry coil in a first mode can function to send intracardiac EGM (IEGM) cardiac signals and wirelessly communicates with at least one of an external device or second implanted device and detects a time varying MR generated gradient electromagnetic field along an axis. In a second mode, the telemetry coil may only send IEGM cardiac signals and wirelessly communicate with at least one of an external device or second implanted device. When used herein, time varying MR generated gradient electromagnetic field include, but is not limited to gradient electromagnetic fields and derivatives of gradient electromagnetic fields. In each example, a first mode and second mode are provided.

The term "discriminate", "discriminated," or "discriminating" refer to the act of distinguishing through determination the identification of two different items. For example, in the context of two signals, the act is to identify the first signal and the second signal and determine that the first signal and second signal are different. In another example, when a first signal presents a cardiac activity signal, a second signal presents a signal generated by a magnetic resonance generated gradient electromagnetic field, the first and second signals are discriminated when the cardiac activity signal is identified, and the MR generated gradient electromagnetic field is identified and a determination is made that two separate signals are provided. Such determination is made in one embodiment if the expected cardiac activity signal contains the presence of RF and/or gradient induced noise.

The term "medical condition" refers to any and all disease, illness, injury, or the like related to a patient's health, organs, metal state, or the like. Medical conditions related to a patient's heart can include ventricular fibrillation (VF), arrhythmia, stroke, heart failure, heart attack, irregular heartbeat, rapid heart rate, high blood pressure, congenital heart disease, heart valve disease, atherosclerosis, pericarditis, etc.

The terms "high frequency" and "HF," as used in connection with pacing pulses and pacing therapy refer to delivering pacing pulses at a rate greater than a rate associated with anti-tachycardia pacing, namely at a rate of at least 30 Hz.

The terms "processor," "a processor," "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

Embodiments may be implemented in connection with one or more implantable medical devices (IMDs). Non-limiting examples of IMDs include one or more of neurostimulator devices, implantable cardiac monitoring and/or therapy devices. For example, the IMD may represent a cardiac monitoring device, pacemaker, cardioverter, cardiac rhythm management device, ICD, neurostimulator, leadless monitoring device, leadless pacemaker, an external shocking device (e.g., an external wearable defibrillator), and the like. For example, the IMD may be a subcutaneous IMD that includes one or more structural and/or functional aspects of the device(s) described in U.S. Patent 10,765,860 titled "Subcutaneous Implantation Medical Device With Multiple Parasternal-Anterior Electrodes"; U.S. Patent 10,722,704, titled "Implantable Medical Systems And Methods Including Pulse Generators And Leads"; US Patent 11,045,643, titled "Single Site Implantation Methods For Medical Devices Having Multiple Leads". Additionally or alternatively, the IMD may include one or more structural and/or functional aspects of the device(s) described in U.S. Patent 9,333,351 "Neurostimulation Method and System to Treat Apnea" and U.S. Patent 9,044,710 "System and Methods for Providing A Distributed Virtual Stimulation Cathode for Use with an Implantable Neurostimulation System". Additionally or alternatively, the IMD may include one or more structural and/or functional aspects of the device(s) described in U.S. Patent Publication No. 2024/0050738 "Active Implantable Medical Device". Further, one or more combinations of IMDs may be utilized from the above mentioned patents and applications in accordance with embodiments herein.

Additionally or alternatively, the IMD may include one or more structural and/or functional aspects of the device(s) described in U.S. Patent 9,216,285 "Leadless Implantable Medical Device Having Removable and Fixed Components" and U.S. Patent 8,831,747 "Leadless Neurostimulation Device and Method Including the Same". Additionally or alternatively, the IMD may include one or more structural and/or functional aspects of the device(s) described in U.S. Patent 8,391,980 "Method and System for Identifying a Potential Lead Failure in an Implantable Medical Device"; U.S. Patent 9,232,485 "System and Method for Selectively Communicating with an Implantable Medical Device"; U.S. Patent 5,334,045 "Universal Cable Connector for Temporarily Connecting Implantable Leads and Implantable Medical Devices with a Non-Implantable System Analyzer"; U.S. Patent 10,729,346, titled "Method And System For Second Pass Confirmation Of Detected Cardiac Arrhythmic Patterns"; U.S. Patent 11,020,036, Titled "Method And System To Detect R-Waves In Cardiac Arrhythmic Patterns"; U.S. Patent 10,874,322, titled "Method And System To Detect Premature Ventricular Contractions In Cardiac Activity Signals"; and U.S. Patent 10,777,880, titled "Adjustable Antenna System to Communication with Implantable Medical Device and Method for Using the Same"; U.S. Patent 9,949,660 titled, "Method and System to Discriminate Rhythm Patterns in Cardiac Activity".

Provided is an IMD and method that, when in an automatic MRI mode for detecting an MRI produced magnetic field in an environment, provides an additional discrimination feature to distinguish between magnetic fields (e.g., static magnetic fields, RF and/or gradient electromagnetic fields, etc.) generated by an environmental magnet such as a handheld magnet and the MRI machine. Upon detection of a magnetic field reaching an initial threshold by a first sensor the mode of operation of the IMD automatically switches from a non-magnetic field (e.g., normal) operating mode to a first magnet mode. The first magnet mode has first settings that assume the magnetic field is created by an environmental magnet. The system provides a first discrimination step by determining whether the strength of the magnetic field that is detected is above a secondary threshold that is greater than the initial threshold. If at or above the secondary threshold, the system automatically switches the IMD to a second magnet mode that has second settings that assume the magnetic field is created by an MRI machine. In one example, when the IMD is in the first magnet mode, the secondary threshold may vary based on the amount of time the IMD remains in the first magnet mode. If the strength of the magnetic field is below threshold, then an additional discrimination step is provided to determine if the magnetic field detected in the environment is generated by an environmental magnet (e.g., handheld magnet) or an MRI machine.

One additional, or alternative, discriminator can be the detection of noise within a cardiac activity signal. A cardiac activity signal, such as an EGM signal, can be recorded by a second sensor (e.g., signal sensing front end) and analyzed to determine whether the time-varying RF or gradient signal generated during an active MRI scan is causing noise within the cardiac activity signal. In one example, the existing noise detection algorithm parameters are varied to optimize for detection of MRI RF or gradient signals in response to detection of the magnetic field. In another example, a noise detection algorithm can define signals outside of that expected from the pace pulse and capture as gradient induced and therefore occurring within the MRI environment.

Another additional, or alternative, discriminator may use the existence of noise in a signal recorded by telemetry circuitry. Noise on a telemetry circuit channel indicates the existence of a time-varying gradient signal generated during an active MRI scan. The detection of this noise may be used to determine the existence of the MRI machine generated magnetic field.

In one example embodiment, an IMD can include both additional discriminators and/or more additional discriminators. In an example the IMD can select between the additional discriminators. Alternatively, each additional discriminator may be used where the detection of a magnetic field formed by an MRI machine by any of the additional discriminators results in the IMD operating in a second magnet mode with settings for an MRI machine environment.

In another example embodiment, the parameters of the first setting are determined such that the treatment, such as a cardiac treatment, provided is safe for a patient if the patient is within an MRI machine environment. By providing parameters that are safe for both an environmental magnet environment and an MRI machine environment, defaulting to the first magnet mode upon detection of a magnetic field in the environment maintains safety for the patient while the additional discriminators are being analyzed.

In another embodiment, the system can self-evaluate the suitability for the MRI scan (such as lead condition, battery status). In an example, the system can use Bluetooth^{®} low energy (BLE) advertising to externally communicate the suitability of the device for an MRI scan.

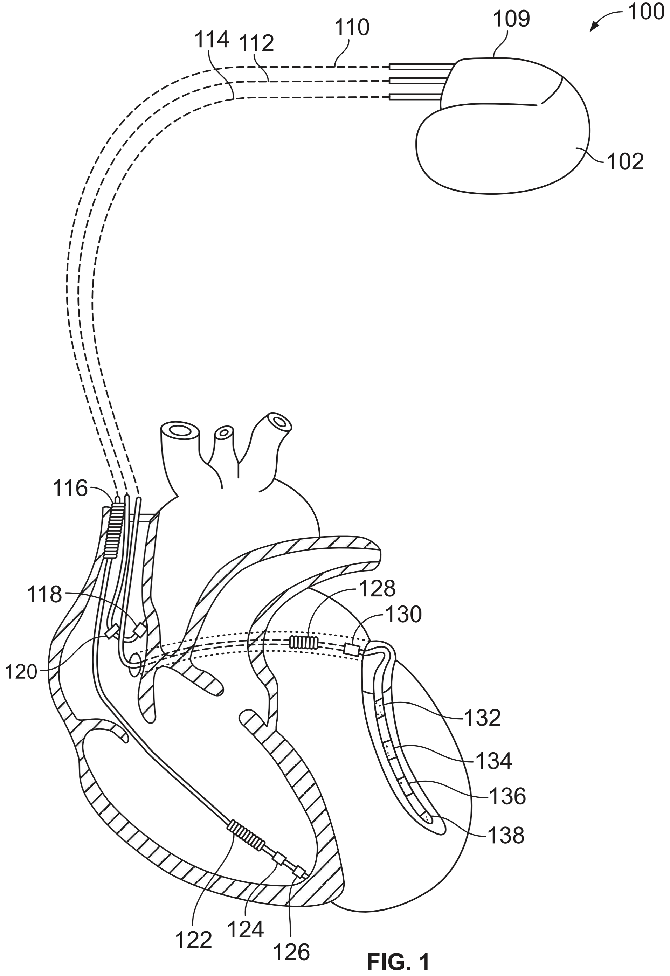

Figure 1 illustrates an IMD 100 that in one example is a dual-chamber stimulation device capable of treating both fast and slow arrhythmias with stimulation therapy, including cardioversion, defibrillation, anti-tachycardia pacing, HF pacing, and pacing stimulation, as well as capable of detecting heart failure, evaluating its severity, tracking the progression thereof, and controlling the delivery of therapy and warnings in response thereto. The IMD 100 may be controlled to sense atrial and ventricular waveforms of interest, discriminate between two or more ventricular waveforms of interest, deliver stimulus pulses or shocks, including LV shocks, MV shocks, and HV shocks, and inhibit application of a stimulation pulse to a heart based on the discrimination between the waveforms of interest and the like. Exemplary structures for the IMD 100 are discussed and illustrated in the drawings herewith.

The IMD 100 includes a housing 102 that is joined to a header assembly 109 that holds receptacle connectors connected to a right ventricular lead 110, a right atrial lead 112, and a coronary sinus lead 114, respectively. The leads 112, 114 and 110 measure cardiac activity (CA) signals of the heart. While the present embodiment is described in connection with CA signals and heart conditions, it is understood that embodiments may be implemented in connection with other types of biological signals and other types of physiologic and pathophysiologic conditions. The right atrial lead 112 includes an atrial tip electrode 118 and an atrial ring electrode 120. The coronary sinus lead 114 includes a left atrial ring electrode 130, a left atrial coil electrode 128 and one or more left ventricular electrodes 132-138 (e.g., also referred to as P1, M1, M2 and D1) to form a multi-pole LV electrode combination. The right ventricular lead 110 includes an RV tip electrode 126, an RV ring electrode 124, an RV coil electrode 122, and an SVC coil electrode 116. The leads 110, 112, 114 detect IEGM signals that are processed and analyzed as described herein. The leads 110, 112, 114 also delivery therapies as described herein.

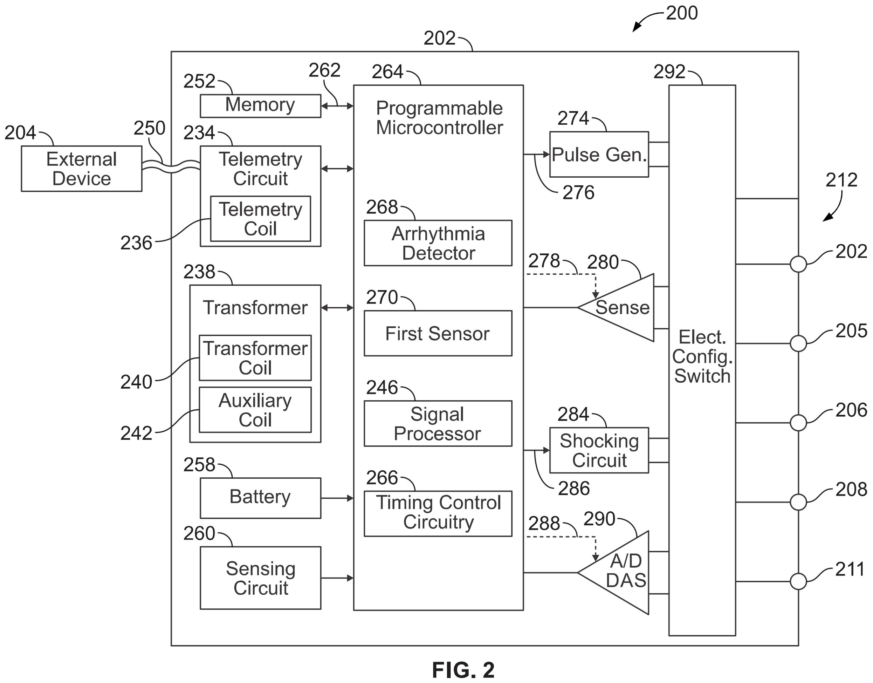

In addition to detecting IEGM signals, the leads 110, 112, 114 can be utilized as sensors for detecting RF and gradient electromagnetic fields that are within the MRI environment of the patient. In one example the leads 110, 112, 114 are configured to detect the gradient electromagnetic field created by an MRI machine. In one example IMD 100 includes a first sensor (first sensor 270 in Fig. 2) and the leads 110, 112, 114 function as a second sensor. The leads 110, 112, 114 are configured to not only detect electrical signals produced by the heart, but additionally to detect the presence of gradient electromagnetic fields within the environment of the IMD 100. For example, when the IMD 100 is within an MRI machine the gradient electromagnetic field produced by the MRI machine can be detected by each individual lead resulting in noise in the CA signals displayed.

Figure 2 illustrates an example block diagram of an IMD 200 that is implanted into the patient as part of the implantable cardiac system. While described as an implantable cardiac system, this is for exemplary purposes only, and other IMDs are contemplated. In this embodiment, the IMD 200 may be implemented as a full-function biventricular pacemaker, equipped with both atrial and ventricular sensing and pacing circuitry for four chamber sensing and stimulation therapy (including both pacing and shock treatment). The pacing circuitry in one example includes HF pacing. Optionally, the IMD 200 may provide full-function cardiac resynchronization therapy. Alternatively, the IMD 200 may be implemented with a reduced set of functions and components. For instance, the monitoring device may be implemented without ventricular sensing and pacing. The IMD 200 of Figure 2 is an example of the IMD 100 shown in Figure 1.

The IMD 200 has a housing 201 to hold the electronic/computing components. The housing 201 (which is often referred to as the "can", "case", "encasing", or "case electrode") may be programmably selected to act as the return electrode for certain stimulus modes. Housing 201 further includes a connector (not shown) with a plurality of terminals 202, 205, 206, 208, and 211 for electrodes (not shown) that provide electronic coupling for electronic circuitry 212 to the electrodes. The type and location of each electrode may vary. For example, the electrodes may include various combinations of ring, tip, coil and shocking electrodes and the like.

The electronic circuitry 212 of the IMD 200 can include a telemetry circuit 234 that as a primary function allows intracardiac electrograms and status information relating to the operation of the IMD 200 (as contained in the microcontroller 264 or memory 252) to be sent to an external device 204 through an established communication link 250. The telemetry circuit 234 wirelessly communicates with at least one of the external device 204 or second implanted device. In addition, the telemetry circuit 234 includes a telemetry coil 236 that may be positioned to detect or obtain a time varying MR generated gradient electromagnetic field along an axis when a patient is undergoing an MRI scan as a secondary function, in addition to communicating the electrograms and status information relating to the operation of the IMD 200. In one example, the telemetry coil 236 is utilized to detect or obtain the time varying MR generated gradient electromagnetic field by using methodologies as described in relation to U.S. Patent No. 8,965,526 to Zhang et al., titled "Active Rejection of MRI Gradient Signals in an Implantable Medical Device". In this manner, the telemetry coil 236 performs dual functions within the IMD 200.

In another example, the telemetry circuit 234 can also function as a second sensor to detect the RF and gradient electromagnetic field of the MRI machine as a result of noise detection. Optionally the telemetry circuit 234 can be utilized to determine the acceptability of the device orientation based on the 3-dimensional microelectromechanical systems (3D MEMS) sensor posture information. The durations of gradient induced signals are typically 200 µs or shorter (the maximum gradient amplitude is 200 ms and many gradient pulses in an MRI sequence are at values less than the maximum), which can be detected by the telemetry circuit 234. While in some embodiments both leads and the telemetry circuit 234 of an IMD 200 can be used as sensors to detect the magnetic field of an MRI, in other examples, such as in leadless devices, only the telemetry circuit 234 is utilized to detect the magnetic field of the MRI machine.

The IMD 200 may, in an embodiment, also include a transformer 238 that includes a transformer coil 240, and an auxiliary coil 242. The transformer 238 as a primary function is configured to at least one of step-up or step-down a voltage between primary and secondary coils 240, 242 to provide power at a select voltage to electronic circuitry 212 of the IMD 200. The transformer coil 240, and auxiliary coil 242 may also be positioned to provide a secondary function of detecting a time varying MR generated gradient electromagnetic field along an axis when a patient is undergoing an MRI. Specifically, when each of the telemetry coil 236, transformer coil 240, and auxiliary coil 242 are positioned at 90° to one another, each axis of a static magnetic field formed by an MRI may be detected and communicated to a microcontroller 264 for analysis to detect the gradient change of the magnetic field. As such, the microcontroller 264 may distinguish between the detected gradient change in the magnetic field.

In the example, electrodes obtain candidate cardiac activity signals in analog form. In one example, the candidate cardiac activity signal is an EGM. The analog candidate cardiac activity signal can then either be passed to an analog-to-digital (A/D) data acquisition system (DAS) 290 coupled to one or more electrodes via a switch 292 to sample candidate cardiac activity signals across any pair of desired electrodes, or to an A/D converter (not shown) of an optional signal processor 246 that receives input from each of the telemetry coil 236, transformer coil 240, and auxiliary coil 242 when functioning to detect the time varying MR generated gradient electromagnetic field along an axis.

The programmable microcontroller 264 controls various operations of the IMD 200. Microcontroller 264 may include a microprocessor (or equivalent control circuitry), random access memory (RAM) and/or read-only memory (ROM) memory, logic and timing circuitry, state machine circuitry, and input/output (I/O) circuitry. The IMD 200 further includes a first chamber pulse generator 274 that generates stimulation pulses for delivery by one or more electrodes coupled thereto. The pulse generator 274 is controlled by the microcontroller 264 via control signal 276. The pulse generator 274 is coupled to the select electrode(s) via an electrode configuration switch 292, which includes multiple switches for connecting the desired electrodes to the appropriate I/O circuits of the electronic circuitry 212, thereby facilitating electrode programmability. The switch 292 is controlled by a control signal from the microcontroller 264.

Microcontroller 264 is illustrated to include an optional timing control circuitry 266 to control the timing of the stimulation pulses (e.g., pacing rate, atrioventricular (AV) delay, atrial interconduction (A-A) delay, or ventricular interconduction (V-V) delay, etc.). Microcontroller 264 also has an optional arrhythmia detector 268 for detecting arrhythmia conditions. Although not shown, the microcontroller 264 may further include other dedicated circuitry and/or firmware/software components that assist in monitoring various medical conditions, including of the patient's heart and managing pacing therapies, including HF pacing.

The microcontroller 264 is also configured to operate in different modes. For example, in one embodiment, the IMD 200 may include electronic circuitry 212 that has dual functionality. Such dual functionality may include a primary functionality for day-to-day activities of a patient, while a secondary functionality may be provided when the IMD 200 is in the presence of a magnetic field. For example, in a non-magnet mode, the electronic circuitry 212 can include components that sense biological signals, deliver cardiac treatment, deliver treatment for a pathophysiologic condition or physiologic condition, wirelessly communicate with at least one of an external device 204 or second implanted device, etc. Meanwhile, in a first magnet mode, the same electronic components of the electronic circuitry 212 add the functionality of detecting the presence of a strong magnetic field.

In one example the electronic circuitry 212 can communicate with a first sensor 270, such as a Hall effect sensor to determine the existence of the magnetic field. The electronic circuity 212 can also determine whether the magnetic field is a weak magnetic field, such as one generated by an environmental magnet (e.g., handheld magnet), or a strong magnetic field such as one generated by an MRI machine. In the first magnet mode the IMD 200 may have a first setting that includes parameters to account for the presence of the weak magnetic field. In one example the parameters are provided so that if the IMD 200 is in an undetected strong magnetic field (e.g., within an MRI machine) that the treatment provided is safe for both the weak magnetic field and strong magnetic field environment.

The electronic circuity 212 can also be configured to have a second magnet mode. The electronic circuitry 212 upon detecting a strong magnetic field or a secondary discriminator can switch the mode of operation of the IMD 200 from the first magnet mode to the second magnet mode. In one example, the strong magnetic field is detected by an additional Hall effect sensor. In this example a first sensor 270 is a Hall effect sensor that detects the existence of a magnetic field in the environment, and a second Hall effect sensor detects the strength of the magnetic field. Upon detection of a magnetic field at or above a secondary threshold, the IMD 200 switches the mode of operation from the first magnet mode to the second magnet mode.

In another example, a different or secondary sensor in the environment is used. For example, the leads (leads 110, 112, 114 in Figure 1) of the IMD 200 can function as a secondary sensor by determining the amount of noise provided in a CA signal. If the amount of noise in the CA signal exceeds a threshold, then an MRI environment is identified (e.g., the presence of an MRI machine is identified) and the IMD 200 switches the mode of operation from the first magnet mode to the second magnet mode. In another example a telemetry circuit 234 functions as a second sensor that determines the amount of noise within a communication channel. If the amount of noise detected exceeds a threshold, again a determination is made that the IMD 200 is within an MRI environment. Upon detection of the MRI environment, the electronic circuitry 212 switches the IMD 200 mode of operation from the first magnet mode to the second magnet mode. In the second magnet mode a second setting with second parameters are provided that is different than the first parameters and first setting. Based on the second settings the IMD 200 delivers treatment, including cardiac treatment, for a pathophysiologic condition or physiologic condition. In an example, in the second magnet mode the electronic circuitry 212 of the IMD 200 functions to treat the patient during an MRI scan.

The IMD 200 also includes a first sensor 270. In one example, the first sensor 270 can be configured to detect a static magnetic field, such as one produced by an MRI machine, or one produced by an environmental magnet, within the environment. In example embodiments the first sensor 270 can be primarily configured to a detect static magnetic field in an environment while other components of the IMD 200 are configured to function as a second sensor that can be also used to detect an MRI environment by detecting the existence of magnetic fields in the environment. In an example the second sensor can include the leads (for instance leads 110, 112, 114 of Fig. 1), or alternatively the second sensor can include the telemetry circuit 234. In each example the second sensor can be configured to have a primary function that does not include detecting an MRI environment, but instead has a secondary function of being utilized to detect or respond to a MRI environment.

In an example, the first sensor 270 is a Hall effect sensor that detects magnetic fields within an environment. In yet another example, the first sensor 270 is a three-dimensional Hall effect sensor. The first sensor 270 may be utilized to detect environmental data utilized to determine if the patient is in an MRI environment with a strong magnetic field. In another example, the first sensor 270 can determine the strength of the magnet field. Upon detection of the static magnetic field, the microcontroller 264 can switch the IMD mode of operation from a non-magnet mode of operation to a magnet mode to treat the patient based on the detection of the magnetic field. In one example, a Hall effect sensor or other sensor configured to detect a magnetic field can be a first sensor 270, while leads or the telemetry circuit 234 used to detect the RF and/or gradient electromagnetic field being generated by an MRI machine is a second sensor.

The IMD 200 may further be equipped with a communication modem (modulator/demodulator) to enable wireless communication with other devices, implanted devices, and/or external devices 204. The IMD 200 also includes sensing circuitry 280 selectively coupled to one or more electrodes that perform sensing operations, through the switch 292, to detect the presence of cardiac activity.

The output of the sensing circuitry 280 is connected to the microcontroller 264 which, in turn, triggers or inhibits the pulse generator 274 in response to the absence or presence of cardiac activity. The sensing circuitry 280 receives a control signal 278 from the microcontroller 264 for purposes of controlling the gain, threshold, polarization charge removal circuitry (not shown), and the timing of any blocking circuitry (not shown) coupled to the inputs of the sensing circuitry.

In the example of Figure 2, a single sensing circuit 280 is illustrated. Optionally, the IMD 200 may include multiple sensing circuit, similar to sensing circuit 280, where each sensing circuit is coupled to one or more electrodes and controlled by the microcontroller 264 to sense electrical activity detected at the corresponding one or more electrodes. The sensing circuit 280 may operate in a unipolar sensing configuration or in a bipolar sensing configuration.

The microcontroller 264 is also coupled to a memory 252 by a suitable data/address bus 262. The programmable operating parameters used by the microcontroller 264 are stored in memory 252 and used to customize the operation of the IMD 200 to suit the needs of a particular patient. Such operating parameters define, for example, pacing pulse amplitude, pulse duration, electrode polarity, rate, sensitivity, automatic features, HF pacing features, arrhythmia detection criteria, and the amplitude, waveshape and vector of each shocking pulse to be delivered to the patient's heart within each respective tier of therapy. Such shocking pulse can be an LV shock, MV shock, HV shock, etc.

A battery 258 provides operating power to all of the components in the IMD 200. The IMD 200 optionally includes an impedance measuring circuit 260, which can be used for many things, including: lead impedance surveillance during the acute and chronic phases for proper lead positioning or dislodgement; detecting operable electrodes and automatically switching to an operable pair if dislodgement occurs; measuring thoracic impedance for determining shock thresholds; detecting when the device has been implanted; measuring stroke volume; and detecting the opening of heart valves; and so forth. The impedance measuring circuit 260 is coupled to the switch 292 so that any desired electrode may be used. The IMD 200 can be operated as an ICD device, which detects the occurrence of an arrhythmia and automatically applies an appropriate electrical shock therapy to the heart aimed at terminating the detected arrhythmia. To this end, the microcontroller 264 further controls an optional shocking circuit 284 by way of a control signal 286, including for LV shocks, MV shocks, HV shocks, etc.

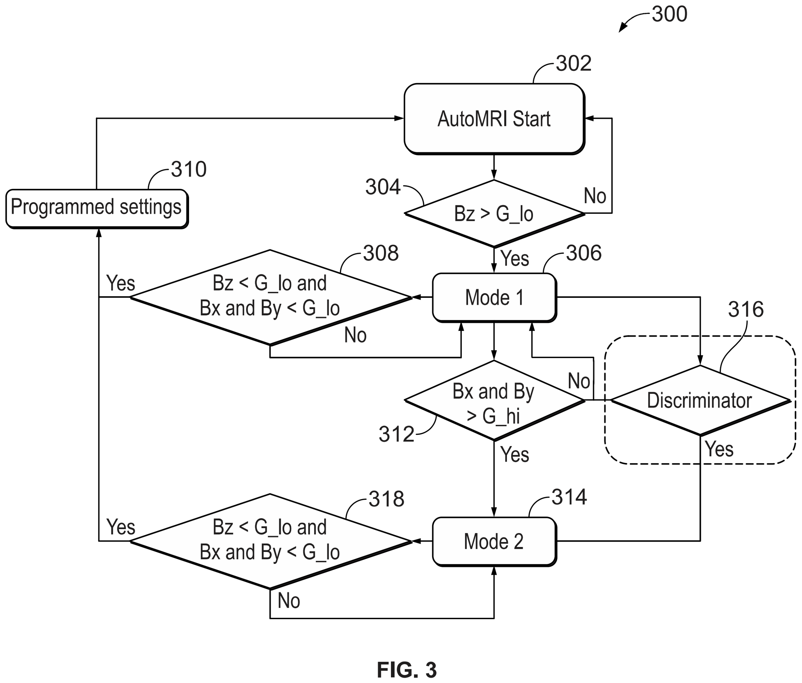

Figure 3 illustrates a method 300 of operating an IMD based on magnetic fields detected within the environment of the IMD. In one example the method can be performed by at least one of the IMDs as described in relation to Figures 1 and 2. In another example the method may be partially performed by at least one of the IMDs or components of the IMDs of Figures 1 and 2. The method 300 can result in a change in the mode of operation of the IMD where the IMD provides a treatment to the patient. In one example the treatment is a cardiac treatment. By changing, modifying, varying, etc. the mode of operation patient treatment is enhanced. In Figure 3, the values Bx, By, and Bz are illustrated and represent the magnetic field detected by a 3D Hall effect sensor along an X-axis (Bx) of the sensor, the magnetic field detected by a 3D Hall effect sensor along a Y-axis (By) of the sensor, and the magnetic field detected by a 3D Hall effect sensor along a Z-axis (Bz) of the sensor.

At 302, one or more processors are placed in an operating condition to detect environmental data in the environment of the IMD. In one example the IMD includes a sensor configured to detect the existence of a magnetic field in the environment. In an example the sensor can be a Hall effect sensor, a 3D Hall effect sensor, giant magnetoresistance (GMR) sensor, or the like. In one example the magnetic field can be a weak magnetic field generated by a magnetic source such as an environmental magnet. In one example the environmental magnet can be a handheld magnet. In another example the magnetic field can be a strong magnetic field and generated by a magnetic source such as an MRI machine.

At 304, the one or more processors determine if a magnetic field (Bz) detected is greater than an initial threshold. In one example the initial threshold is 15 G. In another example the initial threshold is 10 G. In yet another example the threshold is 100 G. If the initial threshold is not met, the one or more processors continue to monitor for a static magnetic field.

If at 304 an initial threshold magnetic field is detected, then at 306 the one or more processors begin operating the IMD in a first magnet mode (mode 1). In one example, in the first magnet mode the IMD has first magnet settings that include parameters based on an environmental magnet being in the environment. In an example the parameters are also determined and based on the environment including a strong magnetic field in the environment such as when the IMD is within an MRI machine. By having the parameters account for both a weak magnetic field (i.e., from an environmental magnet) and strong magnetic field (i.e., from an MRI machine) increased safety is provided.

At 308 while the IMD is operating in the first magnet mode a determination is continuously made whether the strength of the magnetic field (Bz) falls below the initial threshold. If not, the IMD continues to operate in the first magnet mode. If the strength of the magnetic field falls below the initial threshold, then at 310, the one or more processors switch the operating mode from the first magnet mode to a non-magnet mode with determined programmed settings.

At 312, while operating in the first magnet mode, the one or more processors determine if the magnetic field reaches a secondary threshold (G_hi). In one example the first sensor, or same sensor used to detect the initial threshold can be used to determine if the secondary threshold is reached. Alternatively, an additional, or auxiliary sensor such as an additional Hall effect sensor, a 3D Hall effect sensor, GMR or the like can be utilized to determine the secondary threshold is reached. In one example the first sensor is a Hall effect sensor that can be utilized to determine the strength of a magnetic field in an environment of the IMD. In an example the secondary threshold can be 200 G. In another example the secondary magnetic threshold can be 1000 G. In one example, the secondary threshold may vary based on the amount of time the IMD remains in the first magnet mode. For example, the initial secondary threshold can be 1000 G, but after a determined amount of time, such as five minutes, the secondary threshold may decrease to 500 G, then after ten minutes the secondary threshold may decrease again to 200 G. In another example the secondary magnetic threshold can be based on the expected strength of a magnetic field generated by an MRI machine. If at 312 the secondary magnetic threshold is met, then at 314 the one or more processors switch the mode of operation of the IMD from the first magnet mode to a second magnet mode (mode 2). In one example, in the second magnet mode second magnetic settings are provided where parameters can be based on operation of the IMD when in a magnetic field generated by an MRI machine.

If at 312 the secondary threshold is not met, then at 316 the one or more processors utilize an additional discriminator to determine whether the IMD is in an MRI environment such as when within an MRI machine. The additional discriminator can be any additional reading, consideration, or the like that is based on an additional sensor, or not on the first sensor at 312, which detects the presence of MRI environment (e.g. RF and gradient electromagnetic fields). By using a different method or discriminator, if the first sensor cannot detect the strength of the magnetic field for any reason, the additional discriminator verifies whether an MRI device is present in the environment and undetected by the first sensor.

In an example, the additional discriminator is determined using an EGM noise detection algorithm. The leads of the IMD can function as a second sensor that detects not only the electrical signals produced by the heart, but additionally noise in the environment generated by an MRI machine. In one example, for individuals noise windows of time of a heart cycle, the IMD monitors for the determined number of noise detections in each noise window. When the determined number of noise detections are detected, at 314 the one or more processors switch the operating mode of the IMD from the first magnet mode to the second magnet mode. In each operating mode the IMD treats the patient. In one example the treatment is a cardiac treatment. By changing the treatment of the patient to account for the magnetic field in an environment, the treatment and health effects on the patient are improved and enhanced.

In an example, when using an EGM noise detection algorithm as the additional discriminator at 316 the noise caused by RF and/or gradient induced signals can be on all EGM channels whereas a ventricular arrythmia (e.g., heart electrical signal) is only on a ventricular EGM channel. In examples this is the case when the IMD is a dual rhythm (DR) or CRT system. In such an example by using the noise windows additional confirmation or verification is provided that the noise is due to a RF and/or gradient electromagnetic field in the environment such as one generated by an MRI machine, and not an arrythmia. When using the different noise windows, if a similar number of counts, such as +/- 2 counts are detected in each window that noise can be interpreted as due to an MRI machine or strong magnetic field in the environment of the IMD. If at 316 a strong magnetic field is detected, the method would move to step 314 and the IMD operates in the second magnet mode accordingly.

With reference back to step 316, in another embodiment an additional discriminator can be an EGM algorithm that defines any signal outside of an expected signal from a pace pulse and capture. In the example a fixed threshold can be provided that does not vary with time. In an example the fixed threshold can be 2 mV. The IMD can monitor for a determined amount of sensing events, such as three or more sensed events per a cardiac cycle. In an example when the threshold is reached the IMD can be switched to the second magnet mode at 314.

With reference back to the discrimination step at 316, in another embodiment the one or more processors can use an EGM noise detection algorithm to perform an EGM waveform comparison before and after the detection of the magnetic field at step 304. In one example if the rectified signal average is greater than an average threshold, the noise can be interpreted as due to a RF and/or gradient electromagnetic field such as one generated by an MRI machine. In one example the average threshold can be 10%. In another example, a comparison between chambers can similarly be made to determine differences to verify that the difference is not being caused by an electrical signal from the heart. In an example a noise window can be provided for each chamber of the heart and the average threshold must be exceeded in at least two different windows for a determination of a strong magnetic field to be made. In an example, when verification is provided, the IMD switches the operating mode from the first magnet mode to the second magnet mode at step 314.

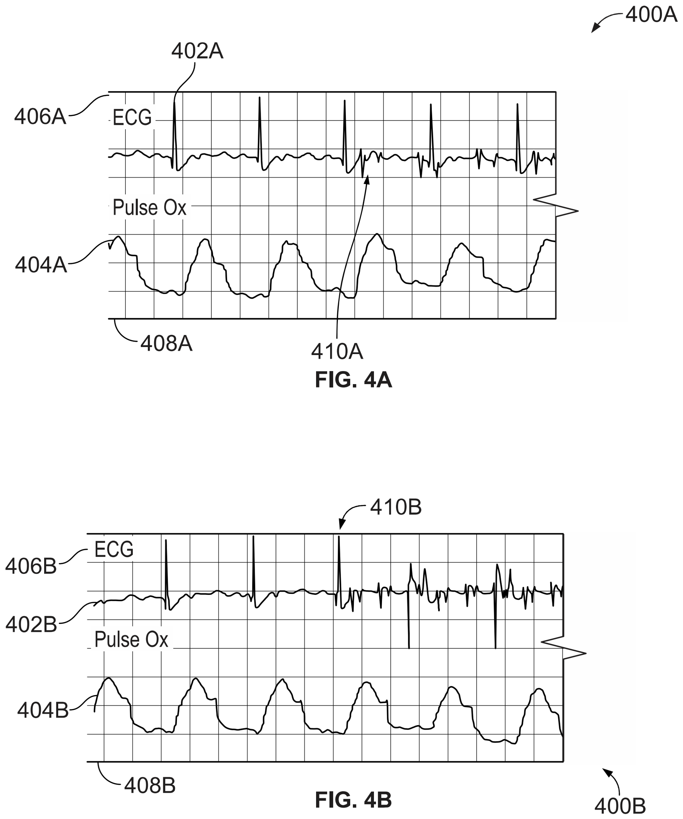

In one example, as indicated above, the additional discriminator can be obtained using a second sensor that includes one or more leads of the IMD. IMDs can include leads that are used to measure the electrical signals of the heart to produce CA signals that can be displayed such as within an electrogram (EGM). Figures 4A and 4B illustrate example electrograms 400A, 400B that includes an electrocardiogram (ECG) 402A, 402B and an oximeter 404A, 404B for detecting oxygen rate. In an example the EGM measures voltage 406A, 406B over time 408A, 408B. In the Figures 4A and 4B, the IMD that provides the measurements for the EGMs 400A, 400B enters into an MRI machine at a start time 410A, 410B. In one example the leads can also be configured to detect environmental data related to RF and/or gradient electromagnetic field within the environment of the IMD. As illustrated, after and during the time the IMD is within the presence of the RF and/or gradient electromagnetic field of the MRI machine, noise can be detected in the ECG 402A, 402B measurements.

In one example an EGM noise detection algorithm can be provided that includes parameters that detect the time-varying gradient and radio frequency (RF) signal generated during an MRI scan. In one example, the gradient and RF induced voltages of the pacing leads are expected to exceed a determined threshold, such as 2 mV, for clinical landmarks. In an example to determine the determined threshold noise detection parameters can include pace refractory and pacing at a determined rate. In one embodiment the determined rate can the 85 beats per minute (bpm) upon detection of the static magnetic field while the determined threshold is 2 mV.

In one example, a detection parameter can include noise window length to account for when the determined threshold is exceeded as a result of electrical activity of the heart. Heart-based electrical activity is only provided in a single chamber or area of the heart and thus does not result in exceeding the determined threshold multiple times in multiple noise windows during a cardiac cycle. By having plural noise windows verification can be provided that the electrical signal that exceeds the determined threshold is as a result of an exterior magnetic field in the environment and not a heart-based electric signal. In one example a noise window can be approximately 62.5 ms.

In another example the number of times the determined threshold is exceeded in a noise window can be parameter. For example three polarity changes for a first noise window and three polarity changes for a second noise window can be required. For fast image acquisition, 128 phase encoding steps can be acquired in less than 20 ms to generate more than 15 polarity changes in the noise window length. MRI sequences with a weighted time constant (T2) can have much longer times (e.g., more than 2 seconds) resulting in only four counts in a 62.5 ms noise window. The number of times the determined threshold is exceeded in a noise window can be determined based on these parameters to ensure accuracy of a detection of noise generated by an MRI machine.

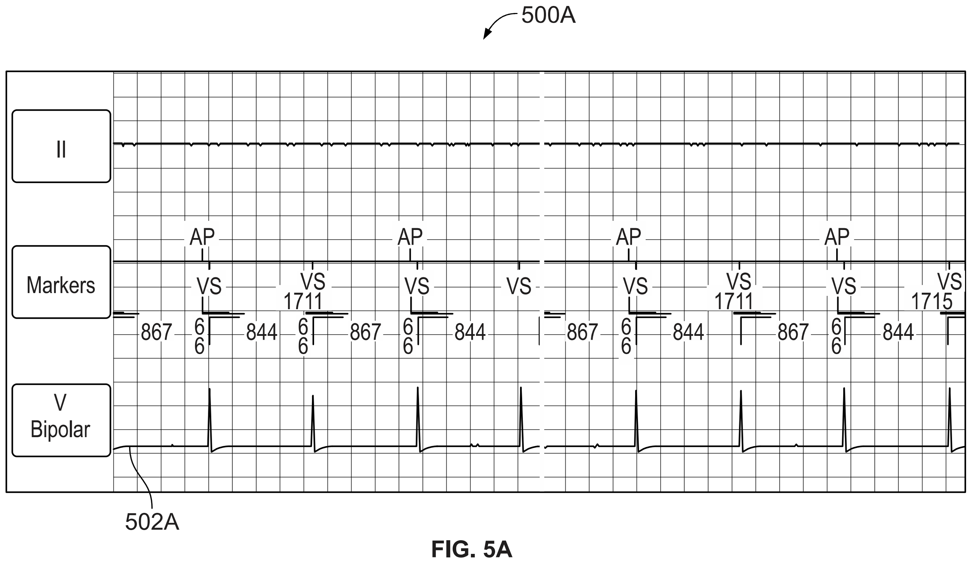

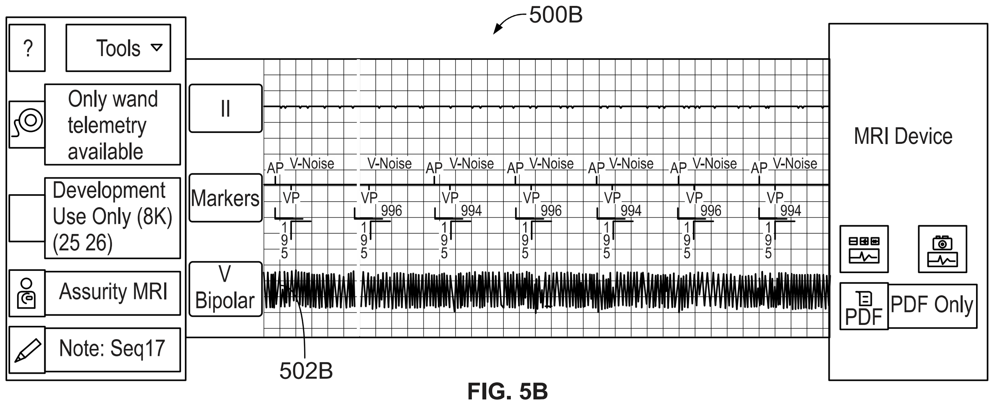

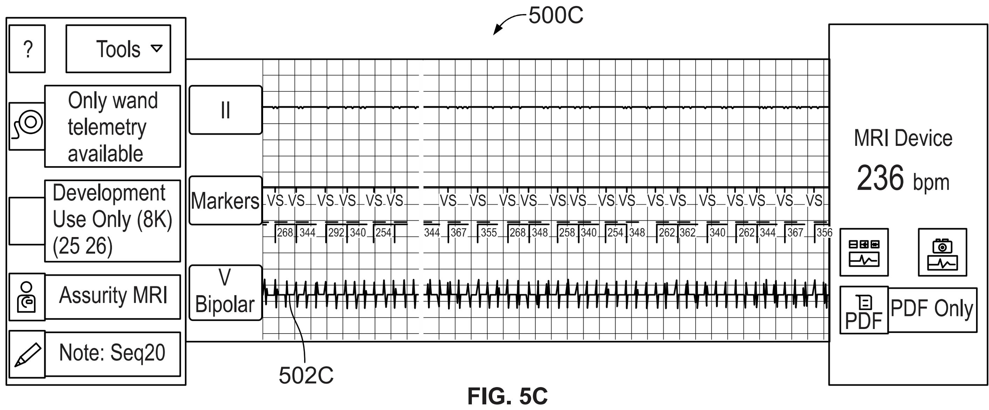

In contrast to Figures 4A-4B, Figures 5A-5C show an alternative EGMs 500A, 500B, 500C used for detecting an MRI gradient electromagnetic field. In this embodiment, Figure 5A illustrates a ventricle bipolar EGM signal 502A when no noise is detected. Figures 5B and 5C illustrate ventricle bipolar EGM signals 502B, and 502C when representative MRI gradient signals 17 and 20 per ISO 10974 are used. Gradient signals 17 and 20 are example gradient signals similar to a localizer scan that occurs at the beginning of an MR scan procedure.

**TABLE 1**

| SeqName | Tslew (ms) | Tdwell (ms) | Toff (ms) | Mag (mV) | nTrain |
|---|---|---|---|---|---|
| Seq17 | 2 | 0 | 5 | 3 | 5 |
| Seq20 | 2 | 0 | 100 | 2.3 | 5 |

Table 1 illustrates characteristics of sequence 17 and sequence 20 wherein Tslew represents the time in which a gradient can be turned off and on measured in ms, Tdwell represents the time interval between digitized samples measured in ms, Toff represents the time interval for a gradient to be turned off, Mag represents the magnitude in mV, and nTrain represents the number of beats in a readout train.

In this example, during sequence 17, three polarity changes for each 62.5 ms noise window would detect an MRI environment. For the sequence 20, the three polarity changes for each 62.5 ms noise window would detect the MRI environment, in part because a localizer is typically 128+ gradient pulses within 15 ms, repeated three times.

In the example when the parameters include rectified average change with a 10% increase indicating an MRI environment, for sequence 17, an MRI environment is detected. For sequence 20, an MRI environment is similarly detected.

With reference back to the discrimination step at 316, in another embodiment, the additional discriminator can also be determined based on the existence of noise in a telemetry circuit of the IMD. In an example, the ratio between the change in amplitude of a magnetic field (dB) and the time it takes to make the change (dt) for a 60 cm gradient coil (of the telemetry circuit of an IMD) scaled to standard resolution (SR)=200T/ms (Teslas per millisecond) is approximately ± 2 T/s. In the example the voltage generated by the telemetry coil is approximately 1 V to 2.35 V/(144 T/s)*(2 T/s) = ± 14 mV (milli Volts) to ± 33mV.

In an example when a first sensor is not in a correct device orientation to maximize induced voltage, the telemetry circuit can be used as an additional discriminator accordingly. In one example a telemetry algorithm can continuously detect if the sensitivity threshold is being exceeded. Alternatively, to save battery life, the telemetry algorithm can be utilized for detection in response to a determination that the first sensor is in an undesirable orientation. In one example this determination can be made using a three-dimensional MEMS sensor that detects posture data and information.

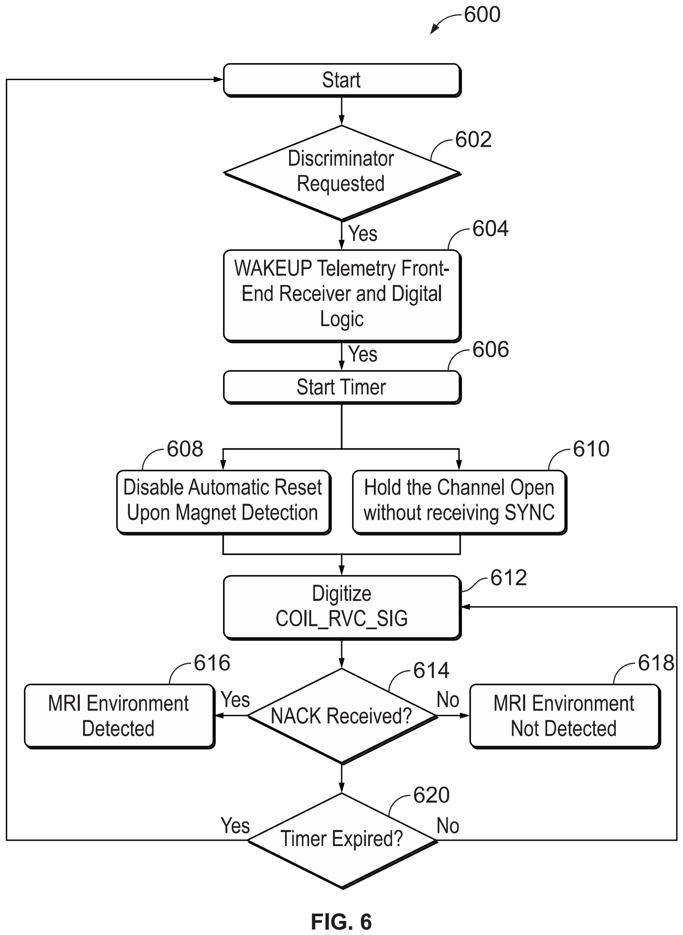

Fig. 6 illustrates and example method 600 for detecting an MRI generated magnetic field with a telemetry circuit. In one example the telemetry circuit is the telemetry circuit as described in relation to Figures 1 and 2. For the avoidance of doubt, the method of Figure 6 is one example of an additional discriminator that can be determined at step 316 of the method of Figure 3.

At 602, the one or more processors determine that an additional discriminator is desired. In one example, the static magnetic field is detected by the first sensor; however, the first sensor does not detect the presence of a strong magnetic field. Upon determining that an additional discriminator is desired, at 604 the one or more processors wake up, or start operating a front-end of the telemetry circuit. In one example upon detection of the static magnetic field the front-end of the telemetry circuit is automatically actuated to begin operating. By having an automatic activation a clinician does not have to be responsible for remembering to change an IMD into the second magnet mode when a patient is going through an MRI machine.

At 606, the one or more processors start a timer for detecting a magnetic field with the telemetry circuit. In one example the magnetic field detected by the telemetry circuit can be an RF and/or gradient electromagnetic field. In one example the timer can be for a determined duration such as ten minutes. In another example the time can be based on the average time for a patient to undergo an MRI scan.

At 608, the one or more processors disable the automatic reset in response to detection of the magnetic field by the first sensor. In one example the automatic reset can be disabled for the determined duration. The method 600 uses the telemetry circuit as a second sensor to detect the magnetic field and not for its primary function for communication. In the example there is not a desire to reset the telemetry circuit upon detection of a communication packet failure because the communication packet failure is what is being used to detect the RF and/or gradient electromagnetic field in the environment of the IMD.

At 610, the one or more processors also hold open a communication channel without receiving SYNC for the determined duration. While the communication channel is held open, at 612 the one or more processors digitize signals received by the telemetry coil. If the digitized signal is the result of RF and/or gradient electromagnetic field in the environment, the created digital signal will be determined to be a failed signal or packet.

At 614, the one or more processors determine whether a non-acknowledgement of the digital signal occurs. If a digital signal is generated as a result of the RF and/or gradient electromagnetic field in the environment, then a non-acknowledgement (NACK) message is generated. The assertion of the NACK bit signifies an invalid communication packet that can be used to identify the presence of the noise in the environment due to the gradient induced voltage of an MRI machine. If a NACK is received then at 616, the one or more processors identify the environment as an environment with an MRI machine. In one example, once the MRI machine is determined or identified to be within the environment, the one or more processors switch the mode of operation of the IMD from the first magnet mode to the second magnet mode at step 314 of Figure 3. If a NACK is not received, then at 618, the one or more processors determine that the RF and/or gradient electromagnetic field has not been detected and digitized by the telemetry circuit and the environment is considered to not have an MRI machine.

At 620, the one or more processors determine whether the determined duration has expired. If not, the one or more processors continue using the telemetry circuit to identify digitized signals resulting from the presence of a strong magnetic field in the environment. Once the timer has expired the method of Figure 6 has ended until restarted by the detection of a magnetic field within the environment.

With reference back to the method of Figure 3, when the IMD operates in the second magnet mode, at 318 the one or more processors determine whether a magnetic field continues to be detected. In one example the first sensor continues monitoring for magnetic fields in the environment of the IMD. If the magnetic field remains, the IMD continues operating in the second magnet mode. In another example, when the first sensor no longer detects a magnetic field that is greater than the magnetic threshold, the one or more processors switch the operating mode of the IMD from the second magnet mode to a non-magnet mode with normally programmed settings that do not account for a magnetic field in an environment of the IMD.

### Closing

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, device, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for conducting operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for conducting the operations and provide this code through its network interface via a network to a second device having a second processor for execution of the code on the second device.

Aspects are described herein with reference to the figures, which illustrate example methods, devices, and program products according to various example embodiments. The program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being conducted in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. An implantable medical device, IMD (200), comprising:
electronic circuitry (212);
a first sensor (270) configured to detect a magnetic field in an environment of the IMD;
a second sensor (110, 112, 114, 234) configured to obtain environmental data;
one or more processors (264); and
a memory (252) coupled to the one or more processors (264), wherein the memory (252) stores program instructions, wherein the program instructions are executable by the one or more processors (264) to:
discriminate between a first magnet mode and a second magnet mode based on the environmental data; and
switch the mode of operation with the electronic circuitry (212) based on discriminating between the first magnet mode and the second magnet mode.

2. The IMD of claim 1, wherein the program instructions are executable by the one or more processors (264) to operate the second sensor (110, 112, 114, 234) with the electronic circuitry (212) in response to detecting the magnetic field.

3. The IMD of claim 1 or 2, wherein the program instructions are executable by the one or more processors (264) to discriminate between the first magnet mode and the second magnet mode by identifying noise caused by radio frequency, RF, and/or gradient electromagnetic field based on the environmental data.

4. The IMD of claim 3, wherein the second sensor (110, 112, 114) is configured to obtain a cardiac activity signal and the noise is within the cardiac activity signal.

5. The IMD of claim 4, wherein the program instructions are executable by the one or more processors (264) to verify the noise within the cardiac activity signal is in response to the RF and/or gradient electromagnetic field.

6. The IMD of claim 5, wherein the program instructions are executable by the one or more processors (264) to verify the noise within the cardiac activity signal by determining whether the noise is within at least two noise windows of the cardiac activity signal.

7. The IMD of claim 3, wherein
the second sensor (110, 112, 114, 234) includes telemetry circuitry (234) configured to transmit communication signals exterior to the IMD (200); and
the noise is detected within the communication signals.

8. The IMD of claim 7, wherein the program instructions are executable by the one or more processors (264) to determine the noise by:
opening, with the telemetry circuitry (234), a communication pathway for a determined amount of time;
digitizing at least one communication packet traveling through the communication pathway;
determining that the at least one communication packet is an invalid communication packet; and
identifying the noise based on the invalid communication packet determination.

9. The IMD of any one of claims 1 to 8, wherein the IMD (200) includes an automatic MRI mode that includes the first magnet mode and the second magnet mode and the program instructions are executable by the one or more processors (264) to operate the IMD (200) in the first magnet mode in response to the first sensor (270) detecting an initial threshold has been exceeded and operate in the second magnet mode when a secondary threshold has been exceeded.

10. The IMD of claim 9, wherein the secondary threshold varies in value based on time operating in the first magnet mode.

11. The IMD of claim 9 or 10, wherein the program instructions are executable by the one or more processors (264) to:
treat a patient based on a first setting when the IMD (200) is within the first magnet mode; and
treat the patient based on a second setting when the IMD (200) is within the second magnet mode.

12. The IMD of any one of claims 1 to 11, wherein the program instructions are executable by the one or more processors (264) to:
switch the IMD (200) into the first magnet mode in response to detecting the magnetic field;
maintain the IMD (200) in the first magnet mode in response to detecting the magnetic field is generated by an environmental magnet; and
switch the IMD (200) into the second magnet mode in response to determining the magnetic field is generated by a magnetic resonance imaging, MRI, machine.

13. A method, comprising:
under control of one or more processors,
detecting, with a first sensor (270), a magnetic field in an environment of an implantable medical device, IMD (200);
discriminating between a first magnet mode and a second magnet mode based on environmental data obtained from a second sensor (110, 112, 114, 234); and
switching the mode of operation with electronic circuitry (212) based on discriminating between the first magnet mode and the second magnet mode.

14. The method of claim 13, wherein discriminating between the first magnet mode and the second magnet mode includes identifying noise caused by radio frequency, RF, and/or gradient electromagnetic field based on the environmental data.

15. The method of claim 13 or 14, wherein discriminating between the first magnet mode and the second magnet mode includes:
opening, with telemetry circuitry (234) of the second sensor (110, 112, 114, 234), a communication pathway for a determined amount of time,
digitizing at least one communication packet traveling through the communication pathway,
determining that the at least one communication packet is an invalid communication packet; and
identifying the noise based on the invalid communication packet determination.
